# EUROPEAN PATENT APPLICATION

(11) **EP 3 545 912 A1**
(43) Date of publication of application: **02.10.2019**
(21) Application number: 19166346.7
(22) Date of filing: 29.03.2019
(51) Int. Cl.: A61F 2/50, A61F 2/76, A61F 2/80

(54) **PROSTHETIC DESIGN**

(30) Priority: 29.03.2018 GB 201805308
(71) Applicant: Open Bionics Ltd, Bristol BS34 8QZ (GB)
(72) Inventor: WOOD, Steve, Swindon, Wiltshire SN2 1LT (GB)
(74) Representative: Bryers LLP

(57) **Abstract**

A method for designing a prosthetic limb including obtaining a three-dimensional model representing a limb of a patient. One or more criteria for a prosthetic limb are determined, from the three-dimensional model. Object data is generated for one or more elements of the prosthetic limb according to the determined criteria.

## Description

The present invention relates generally to methods for designing and fabricating prosthetic limbs, said fabrication being by, for example, additive manufacturing and particularly, although not exclusively, a prosthetic arm.

### Background

A prosthetic arm designed to fit transradial amputees may be comprised of three main subassemblies; the hand; the wrist; and the socket. Additionally, optional, swappable covers can be added to style the arm. The system is actuated by motors concealed within the palm. It is powered by a battery located either just below the elbow or inside the distal end of the arm. The user controls the system by flexing the muscles of their forearm; the system senses these flexes with Electromyographic (EMG) sensors embedded in the socket.

### Summary of Invention

According to an aspect of the present invention there is provided a method for designing a prosthetic limb comprising: obtaining a three-dimensional model representing a limb of a patient; determining, from the three-dimensional model, one or more criteria for a prosthetic limb; and generating object data for one or more elements of the prosthetic limb according to the determined criteria. For example, the three-dimensional model may be obtained by accessing a data file containing the 3D model. The data file may be generated by scanning a limb to which the prosthetic is to be fitted.

The one or more elements may include an outer frame portion of the prosthetic limb. The frame portion may encase an inner portion of the prosthetic limb. A frame configuration may comprise a plurality of frame portions, whereby the object data is for the plurality of frame portions based on the frame configuration.

The one or more elements may include an inner socket portion of the prosthetic limb.

The one or more elements may include a swappable cover arranged to cover at least a portion of the outer frame portion.

The criteria may relate to positions of components of the prosthetic limb. The one or more components comprise any one of: a frame tensioning dial, frame tensioning cabling, Electromyographic (EMG) sensors, EMG sensor cabling, cover attachments, one or more batteries, battery cabling, batter power connection and a socket locking mechanism Determining the position of a component may comprise determining an optimal position for the component based on dimensions of the limb of the patient. Determining a position of a component may comprise determining an optimal position of the component with respect to one or more other components of the prosthetic limb.

The object data may define, for each determined position, opening portions for the respective components in one or more elements of the prosthetic limb.

The object data may determine a planar model of the one or more elements. In other words a flat 2D model which may be 3D printed or otherwise fabricated and then subsequently formed into the desired shape.

In a further aspect, there is provided a method of manufacturing a prosthetic limb comprising fabricating an object according to the object data generated according to the method as set out in any of the above statements.

The method may include fabricating one or more elements of the object using fused deposition modelling. For example, by additive manufacturing process.

In a further aspect of the invention, there is provided a computer program comprising processor executable code which upon execution causes a processor to perform the method as set out in any of the preceding statements.

In a yet further aspect of the invention, there is provided a device configured to perform a method according to any of the preceding statements. The device may comprise a processor and a memory, the memory containing processor executable instructions for performing the method.

According to an aspect of the present invention there is provided an additive manufacturing method for a prosthetic limb comprising: accessing a three-dimensional model representing a limb of a patient; determining, from the three-dimensional model, one or more criteria for a prosthetic limb; and generating object data for an outer frame portion of the prosthetic limb according to the determined criteria.

The "criteria" may be positions of sensors and dimensions of the prosthetic arm.

The "object data" may be CAD file corresponding to the flattened frame portion.

In some embodiments one or more components of the limb, such as an arm, are formed using 3D printing. In some embodiments a selective laser sintering process is used to form some components, such as an inner socket and/or an outer frame.

Different embodiments of the present invention can, for example, operate as follows:
a) start with a model of a patient's arm, generate a model in 3D of the prosthetic arm including the frame portion and components, then generate the "object data" for the flattened frame; or
b) start with a model of a patients arm and then generate "object data" for the flattened frame directly from the model of the patient's arm.

In some ways the present invention can be thought of as transitioning from a 3D phase (e.g. taking a scan from a patient) through a 2D phase (flattening of a pattern) and back to a 3D phase (forming a 2D pattern into a final shape). In some embodiments, for example, the first 3D phase involves determining general characteristics of the required arm e.g. length and wrist diameter; this is converted into a 2D object before further detail is added e.g. cable paths and battery/sensor positions; the 2D object is convertible into a 3D shape i.e. design of the 2D object is constrained so that it can be converted into a 3D form thereafter.

In some embodiments the frame portion encases an inner portion of the prosthetic limb.

The method may further comprise fabricating an object according to the object data.

In some embodiments the object data determines a planar model of the frame portion.

The method may further comprise thermoforming the frame portion from the object.

The method may further comprise fabricating a buck corresponding to the three-dimensional model representing the (desired) limb of the patient including components for the prosthetic limb.

In some embodiments the frame portion is thermoformed around the buck.

In some embodiments the criteria relate to positions of components of the prosthetic limb.

In some embodiments the object data determines an interior of the frame portion including, for each determined position, opening portions for the respective components.

Determining the position of a component may comprise determining an optimal position for the component based on dimensions of the limb of the patient.

Fabricating the frame portion may comprise fabricating the frame portion using fused deposition modelling.

The method may further comprise determining a frame configuration comprising a plurality of frame portions.

The method may further comprise generating the object data for the plurality of frame portions based on the frame configuration.

A further aspect provides an additive manufacturing method for a prosthetic limb comprising: retrieving a three-dimensional model representing a limb of a patient; determining, from the three-dimensional model, criteria for a prosthetic limb; and generating object data for an inner socket portion of the prosthetic limb, based on the criteria.

The method may further comprise fabricating an object according to the object data.

In some embodiments the criteria relate to positions of components of the prosthetic limb.

In some embodiments the object data determines an exterior of the socket portion including, for each determined position, opening portions for the respective components.

A further aspect provides a method of assembling a prosthetic limb comprising: fabricating one or more three-dimensional objects, from object data corresponding to an outer frame portion of the prosthetic limb; thermoforming the one or more objects to form respective outer frame portions of the prosthetic limb; fabricating one or more inner socket portions of the prosthetic limb; and assembling a portion of the prosthetic limb from the one or more inner socket portions and outer frame portions by encasing the one or more inner portions with the outer frame portions.

Assembling the portions may comprise mounting one or more components with the one or more frame portions and socket portions.

In some embodiments the one or more components comprise any one of: a frame tensioning dial, frame tensioning cabling, Electromyographic (EMG) sensors, EMG sensor cabling, cover attachments, one or more batteries, battery cabling, batter power connection and a socket locking mechanism

Different aspects and embodiments of the invention may be used separately or together.

Further particular and preferred aspects of the present invention are set out in the accompanying independent and dependent claims. Features of the dependent claims may be combined with the features of the independent claims as appropriate, and in combinations other than those explicitly set out in the claims.

The following example embodiments are described in sufficient detail to enable those of ordinary skill in the art to embody and implement the systems and processes herein described. It is important to understand that embodiments can be provided in many alternative forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

A further aspect provides a method of manufacturing a robotic prosthetic arm comprising: collecting data relating to a residual arm of a patient; determining, from the data, one or more criteria for a bespoke prosthetic arm; and generating data to facilitate fabrication of one or more components of the prosthetic arm according to the determined criteria.

### Brief Description of the Drawings

Figure 1 shows a socket portion of a prosthetic arm according to a first example;
Figure 2 shows a cross section of a prosthetic arm formed of a frame and socket about a proximal socket end according to the first example;
Figure 3 illustrates cable channels in the socket portion of a prosthetic arm;
Figure 4 shows an exploded view of the prosthetic arm of the first example;
Figure 5 shows an exploded view of a prosthetic arm in a second example;
Figure 6 shows an upper frame portion of the frame in the first example;
Figure 7 shows a lower frame portion of the frame in the first example;
Figure 8 shows a left frame portion in the frame of the second example;
Figure 9 shows a right frame portion in the frame of the second example;
Figure 10 shows a prosthetic arm with an assembled frame having an open core structure;
Figure 11 illustrates a flow chart for a method of designing a prosthetic limb in an embodiment; and
Figure 12 illustrates a device for designing a prosthetic limb in another embodiment.

### Detailed Description

Elements of a prosthetic arm may include a liner (also referred to as a socket) which is encompassed by a frame. According to an example, a socket may be printed in semi flexible Cheetah plastic from Fenner Drives which is a certified medical safe material to ISO 10993, tested by Envigo Laboratory.

Due to the socket's flexibility and design profile, it is both expandable and compressible which allows some growing room and an element of conformality to the user's residual arm shape. Adjustability of the fit comes from the external panels compressing on the outer surface of the socket via a cable tensioning system.

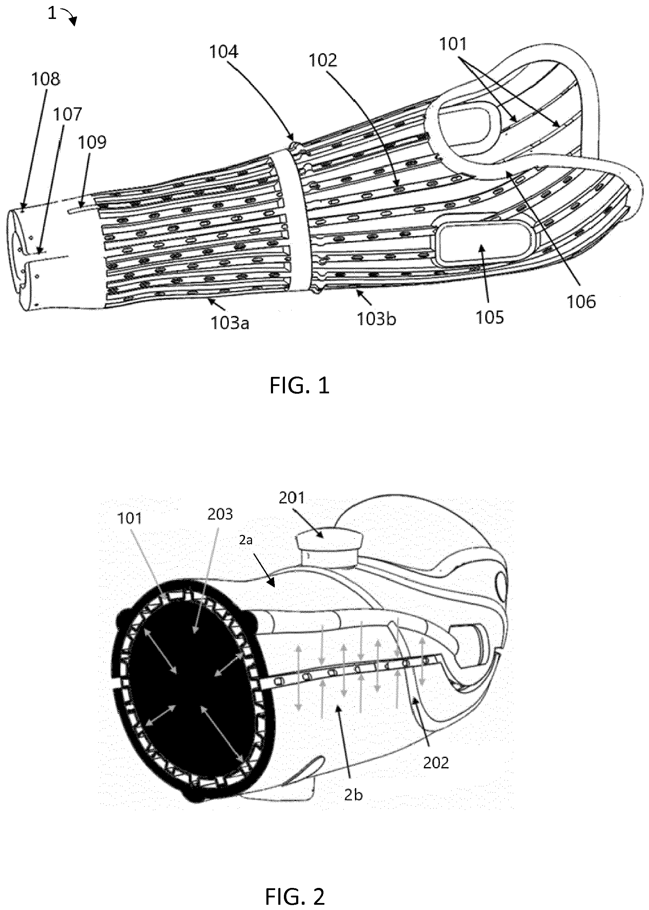

Figure 1 shows the various features on the socket 1. Ventilation is achieved due to the fluted nature of the socket where small air channels have been incorporated, the fluted channels 101 are printed with holes 102 to allow heat and moisture to escape externally and allow fresh air to permeate through to the skin. The covering frames may also be aerated via a mesh like structure which helps reduce heat containment. Figure 2 shows how the covering first and second (upper and lower) frame portions 2a, 2b compress on the flutes 101 via a cable tensioning system comprising a tensioning dial 201 configured to actuate tensioning cable located within frame tensioning cable channels 202 arranged to adjust the amount of tension in the upper and lower frames 2a, 2b that is applied to a residual limb 203. Due to the thin walled nature of the flutes 101, the socket 1 adjusts its diameter to conform to a range of shapes.

The socket is printed in two parts, a fixed distal section 103a which is attached to the wrist via the socket fasteners and a removable proximal section 103b that can be washed and cleaned easily using antibacterial wipes, for example. The double section socket 103a, 103b also makes the 3D printing process more stable by reducing the need to print tall slender flexible objects. The proximal section 103b of the socket is held in place by a locking bead 104 feature which is captivated by the outer frames 2a, 2b coupled with the cable tensioning system 201, 202.

For each patient an optimum EMG sensor position must be attained. The socket has cut-outs 105 shaped so that sensor assembly can be pushed through from the outside to achieve fitment against the skin at the desired location.

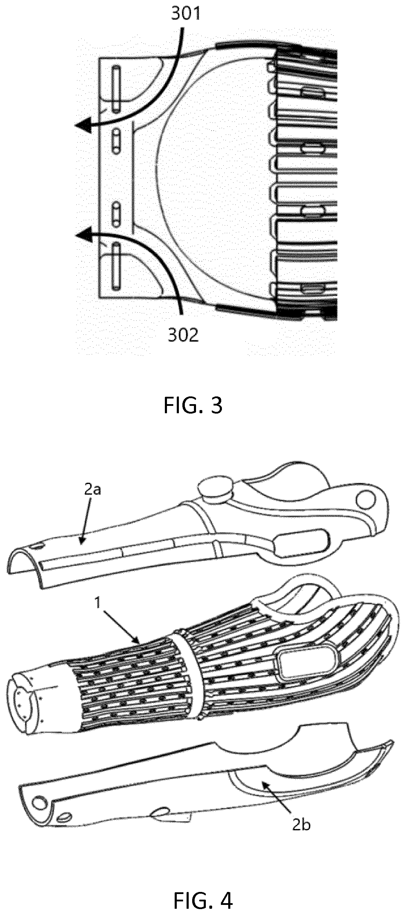

Cable management has to pass through from the outside of the arm, through the outer frames and socket into the wrist. This is made possible by cable entry slots 107 in the wrist portion of the distal socket 103a. The distal end of the socket has first and second (upper and lower) cable channels 301, 302 for the EMG and battery power cables to pass through as shown in Figure 3.

The flared entry (socket flare) 106 around the elbow has been extended to cover the epicondyle areas to achieve some clamp and prevent the socket from falling off. During the scan rectification phase, these areas can be reworked to give extra clamp. These areas on the clamping frames can also be reworked with heat at the patient fitment phase. Running along the length of the socket are location ridges 109 for the outer frames, this is to stop any radial slip during the tightening process with a cable tensioning system. Around the wrist of the distal socket 103 are provided wrist fastener holes 108 which are configured to received complimentary self-tapping fasteners of the first and second frame portions 2a, 2b.

External to the socket are two large frames 2a and 2b that provide an adjustable clamping force to retain the socket on the arm by way of the cable tensioning system mentioned above.

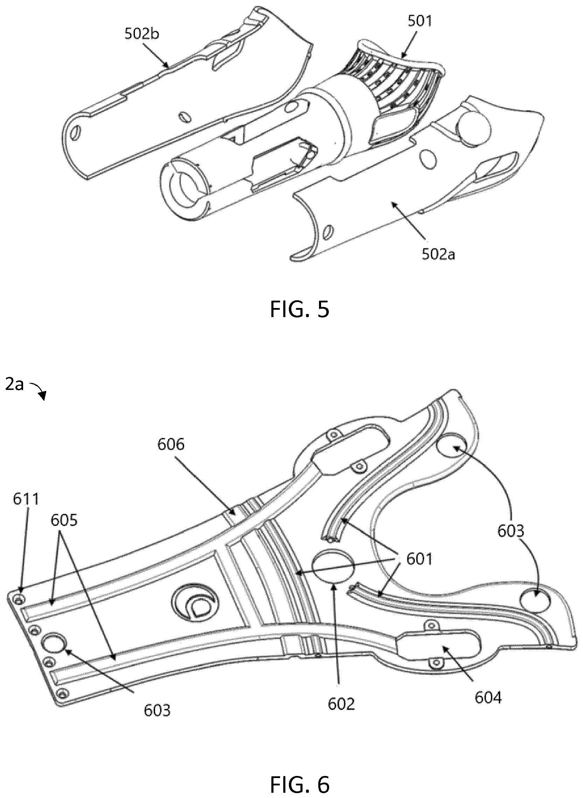

First and second configurations of the arm is contemplated, which impacts the shape of the frames. One configuration is to have the battery pack attached externally to the arm and for this we split the frames into an upper and lower configuration as shown in Figure 4. The second configuration is to have the battery internal to the distal end of the arm socket 501 and for this we split the frame into a left and right frame portions 502a, 502b configuration (left and right in this sense being generally with respect to the orientation of the prosthetic when in use from the perspective of the weather, as will be appreciated other orientations are possible) as shown in Figure 5.

The frames are attached to the distal end of the socket by four self-tapping fasteners (not shown) in each frame.

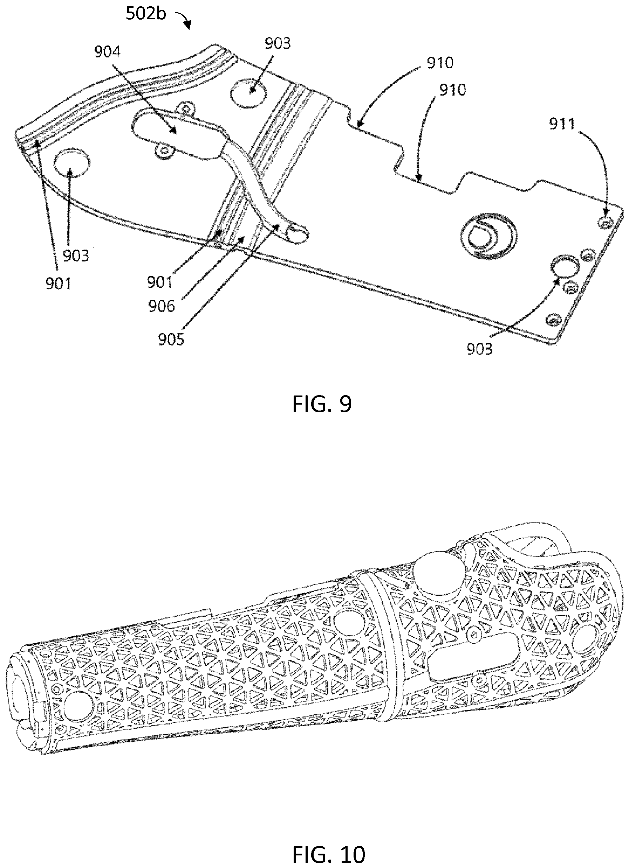

In an example the frames are designed to be 3D printed flat and then thermoformed with heat to their desired shape on a 3D printed buck. An example of a thermoformed frame can be seen in Figure 10. This method achieves quick 3D print time and much stronger part due to the lamina direction. Forming a flat frame creates a lamina flow which follows the contours of the arm in a similar manner that a forged part creates a flowing grain direction that follows the shape of a component. To aid the thermoforming process, the forming buck is 3D printed, which is a copy of the patient's arm scan with some extra features. Location features are modelled into the buck to align important details in the frames such as EMG sensors, Tensioner and cover attachment locations. However, the elements of the prosthetic may be printed directly from an object data file in 3D form ready and assembled.

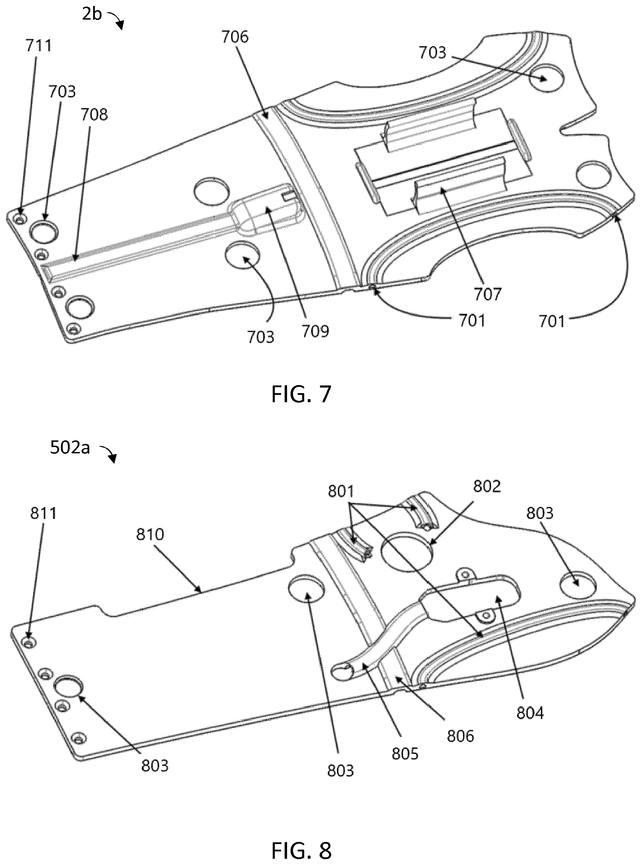

Figures 6 and 7 show upper and lower frame portions 2a and 2b when 3D printed flat and Figures 8 and 9 show respective left and right frame portions 502a, 502b when 3D printed flat.

Throughout the proximal section of the frame portions 2a, 2b, 502a, 502b are a series of channels 601, 701 801, 901 that allow the cables of the tensioning system (e.g. a Click Medical tensioning system) to pass through, thus joining the two halves of the outer frames together. The tensioner has a printed outer socket 602, 802 which is subsequently friction stir welded into a receiving hole in one of the frames as shown in Figure 6 and Figure 8.

Cover attachment pockets (i.e. holes) 603, 703, 803, 903 are provided for attachment of matched covers to the frame portions. The covers may be purely decorative but could conceivably provide additional protection (e.g. from impacts) to the frame and socket elements of the prosthetic. The cover may protect all or some of the frame. For example, it may be supplied as a short cover which is designed to at least protect a battery assembly in the lower frame portion 2b, where the cover extends longitudinally only across part of the lower frame portion 2b. The covers may be attached by push fit pins that are 3D printed to be integral to the covers. The push fit pins press into an insert which is inserted into the cover attachment pockets.

In each of the frame portions 2a, 2b, 502a, 502b, four frame attachment apertures 611, 711, 811, 911 are provided which are arranged to compliment similar openings on the socket. The frame portions may be thereby attached to the socket via the openings using a fastener (e.g. snap fit or bolted).

EMG sensors may be affixed via the sensor cutouts (apertures) 604, 804, 904 in the upper 2a and left and right 502a, 502b frame portions respectively. Cable routing is provided for via EMG sensor cable channels 605, 805, 905.

A proximal socket locking groove 606, 706, 806, 906 is provided in each of the frame portions 60.

Another feature gained by FDM (Fused Deposition Modelling) printing flat is the ability to print the part without any upper and lower surfaces thus exposing the triangular mesh inside, this is known as "Open Core" as shown in Figure 10. It creates a very lightweight, yet strong and faster to print component that allows a good amount of airflow through the frame to the internal socket. 3D printing this mesh in this way removes the time and complexity of modelling the ventilation in CAD. The mesh allows a bit more stretch and compression when working the frame over the forming bucks during thermoforming, reducing the risk of creasing.

Large flat frames may be split proximal into and distal sub-components to allow them to fit on a 3D printing bed. The proximal and distal frames are joined by a friction stir weld before forming. The location of the joint is subtly hidden in the distal socket locking bead groove

On the underside of the frames are longitudinal grooves which line up with the socket ridges to help alignment during the thermoform phase and tensioning the socket.

In the upper frame portion 2a shown in Figure 7, battery retaining clips 707 are provided to which a battery may be attached thereto. A battery cable channel 708 and battery power connector enclosure 709 are also provided. Where the left and right frame portions 502a, 502b are used, in order to provide battery and battery connector access, a number of cutouts 810, 910 are provided. The cutouts 810, 910 combine to define an aperture when the frame portions are assembled through which the battery and connector can be accessed.

All electrical cable routing is encapsulated in channels 605, 705, 805, 905 printed for the EMG sensors and captivated battery connector.

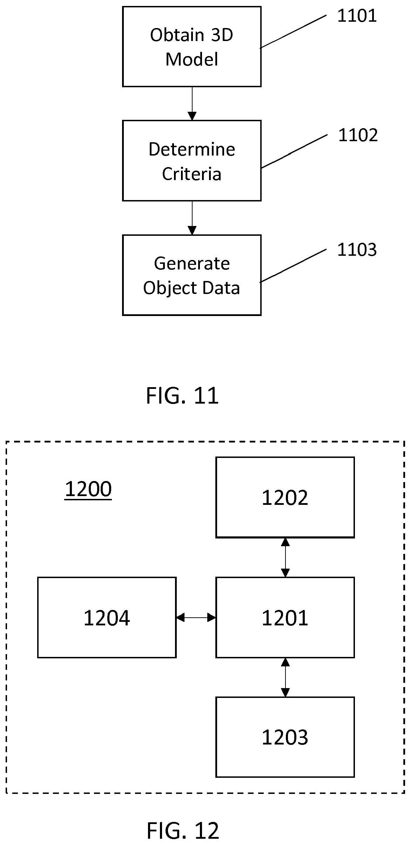

An embodiment of the invention provides a method of designing a prosthetic limb which is performed using a three-dimensional model of a patient's arm. The steps performed are shown in Figure 11. Specifically, in a first step 1101, data representing a three-dimensional model of patient's limb is obtained. In order to design a prosthetic arm, for example, the model obtained might represent the dimensions of a residual arm of a patient for whom the prosthetic is being designed. The model might be obtained by 3D scanning the patient's limb or by taking various measurements of the dimensions of the limb and creating a model based on those measurements.

This model is then utilised to determine in step 1102 criteria for the prosthetic limb to be designed. The model may be a virtual model formed from 3D data and realised via computer aided design software in 3D design environment. This of course may relate to the size and shape of the elements such as the socket and frame so that they are suitable for the patient. For example, the length of a prosthetic arm and the wrist diameter might be important criteria. The criteria may also relate to components of the prosthetic limb such as a frame tensioning dial, frame tensioning cabling, Electromyographic (EMG) sensors, EMG sensor cabling, cover attachments, one or more batteries, battery cabling, batter power connection and a socket locking mechanism. Based on the 3D model, optimal positions for these components might be set as criteria. For example, it might be determined if the battery is better to be on the outside or inside of the frame portions (e.g. the Figure 4 or Figure 5 examples). The location of the EMG sensors might be determined for best positioning relative to the residual arm to provide good signal strength and/or operation e.g. in order to effectively operate a robotic hand or appendage to the prosthetic arm. In turn, the position of the apertures for the EMG sensor might impact whether the battery is inside or outside the frame. Similarly, the position of the tensioning system (dial and tension cables) might be a criteria. Again, the position of the tensioning system components will need to be balanced against the position of other components and their cabling requirements. Accordingly, criteria are set that allow for an optimal position of components. Some of the criteria may be set automatically and others by the designer in the 3D environment, for example, by placing components about the virtualised model of the limb. An example, of a criteria (i.e. design rule) which can be automatically applied might be that the battery harness is a predetermined distance from the elbow.

Once the criteria for the prosthetic have been determined, e.g. positions and dimension of elements and components of the prosthetic determined, object data is created, in step 1103, for one or more elements according to the criteria. Specifically, a 3D data file may be generated in a format that is suitable for export to a 3D printing device. One or more data files may be generated, each corresponding to an element of the prosthetic e.g. one file for the proximal socket, one for the distal socket, one for the upper frame portion, one for the lower frame portion etc. The elements will have dimensions specified according to the criteria based on the 3D model of the limb to which the prosthetic is to be fitted, and be designed, at least in some embodiments, in view of the optimal position of components. For example, if criteria for the EMG sensor opening and battery position are provided, then cable routing channels in the frame and/or socket may be determined to provide cabling to and from the EMG sensor and battery. These channels can be configured in the design so that standard cabling may be used, for example. Conventional routing algorithms may be used to route the cables and channels given the placement criteria of the components and the dimensions of the socket and/or frame. The routing may have to comply with various design rules that determine, for example, that relate to electrical compliance, e.g., proximity of electrical components and permissible cable lengths etc and/or comfort/aesthetic related rules for the prosthetic limb. The generated 3D data file may then be used to 3D print the elements according to the design. The 3D printed elements can be assembled and used to create the prosthetic limb.

Accordingly, a bespoke prosthetic limb design may be created for a patient with very little user input required.

In embodiments where the model to be 3D printed is to be printed flat of the process can, for example, operate as follows:
a) start with a model of a patient's arm, generate a model in 3D of the prosthetic arm including the frame portion and components, then generate the "object data" for the flattened frame; or
b) start with a model of a patients arm and then generate "object data" for the flattened frame directly from the model of the patient's arm.

In such embodiments, the invention effectively applies a method in which there is a transitioning from a 3D phase (e.g. taking a scan from a patient) through a 2D phase (flattening of a pattern) and back to a 3D phase (forming a 2D pattern into a final shape). In some embodiments, for example, the first 3D phase involves determining general characteristics of the required arm e.g. length and wrist diameter; this is converted into a 2D object before further detail is added e.g. cable paths and battery/sensor positions; the 2D object is convertible into a 3D shape i.e. design of the 2D object is constrained so that it can be converted into a 3D form thereafter. In other embodiments the components are formed directly as 3D shapes (i.e. no post-printing forming).

The design process may be performed in software, for example by one or more processors and a memory in which a computer program is stored. Alternatively, the method may be implemented in circuitry of a device. For example, an ASIC, a reconfigurable logic circuit, or implemented using a general-purpose computer. The program may be stored on a machine or computer readable-medium or may be embodied by a signal or data carrier. The computer readable medium may be transitory or non-transitory.

Figure 12 shows a device according to embodiments of the invention. The device 1200 comprises a processor 1201, short-term memory 1202 (e.g RAM), storage 1203 (e.g. hard disc), and input-output I/O interface 1204. The device may be configured to perform the method specified above by having suitable processor implementable instructions stored in the memory 1203 and executed by the processor 1201 in conjunction with the short-term memory 1202. The 3D model data may be obtained from the long-term storage 1203 or be input or derived based on parameters relating to the properties of the patient's limb entered via the I/O interface 1204.

Although in the embodiments described above, reference is made to a prosthetic arm, it will be understood that the invention is applicable to the design and fabrication of other prosthetic limbs, e.g. a prosthetic leg.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims and their equivalents.

## Claims

1. A method for designing a prosthetic limb comprising:
obtaining a three-dimensional model representing a limb of a patient;
determining, from the three-dimensional model, one or more criteria for a prosthetic limb; and
generating object data for one or more elements of the prosthetic limb according to the determined criteria.

2. The method according to claim 1, wherein the one or more elements include an outer frame portion of the prosthetic limb.

3. The method according to claim 2, wherein the frame portion encases an inner portion of the prosthetic limb.

4. The method according to claims 2 or 3, comprising determining a frame configuration comprising a plurality of frame portions, whereby the object data is for the plurality of frame portions based on the frame configuration.

5. The method according to any preceding claim, wherein the one or more elements include an inner socket portion of the prosthetic limb.

6. The method according to any of claims 2 to 5, wherein the one or more elements include a swappable cover arranged to cover at least a portion of the outer frame portion.

7. The method according to any one of claims 1 to 6 wherein the criteria relate to positions of one or more components of the prosthetic limb.

8. The method according to claim 7, wherein determining the position of a component comprises determining an optimal position for the component based on dimensions of the limb of the patient.

9. The method according to claim 8, wherein determining a position of a component comprises determining an optimal position of the component with respect to one or more other components of the prosthetic limb.

10. The method according to claims 7 to 9, wherein the object data defines, for each determined position, opening portions for the respective components in one or more elements of the prosthetic limb.

11. The method according to any preceding claim, wherein the object data determines a planar model of the one or more elements

12. A method of manufacturing a prosthetic limb comprising fabricating an object according to the object data generated according to the method of any of claims 1 to 11.

13. The method according to claim 12, comprising fabricating one or more elements of the object using fused deposition modelling.

14. A computer program comprising processor executable code which upon execution causes a processor to perform the method of any one of claims 1 to 11.

15. A device configured to perform a method according to any of claim 1 to 11.
